# EUROPÄISCHE PATENTANMELDUNG

(11) **EP 3 266 495 A1**
(43) Veröffentlichungstag der Anmeldung: **10.01.2018**
(21) Anmeldenummer: 16178481.4
(22) Anmeldetag: 07.07.2016
(51) Int. Cl.: A61M 39/02, A61N 5/06, A61L 2/08

(54) **EINRICHTUNG ZUR DURCHFÜHRUNG EINER LEITUNG DURCH DIE HAUT EINES PATIENTEN**

(71) Anmelder: Berlin Heart GmbH, 12247 Berlin (DE)
(72) Erfinder: Wiesener, Constantin, 14471 Potsdam (DE)
(74) Vertreter: Pfenning, Meinig & Partner mbB

(57) **Zusammenfassung**

Die Neuerung bezieht sich auf eine Einrichtung zur Durchführung einer Leitung (6, 6', 6'') durch die Haut (5) eines Patienten, wobei die Leitung einen photodynamischen Stoff (8) aufweist, der bei Bestrahlung hochreaktive Sauerstoffderivate freisetzt. Somit kann eine Desinfektion/Entkeimung im Bereich einer Durchführung sowohl auf der Außenseite der Haut als auch im Durchführungsbereich durch die Bestrahlung der Durchführung erreicht werden.

## Beschreibung

Die Erfindung liegt auf dem Gebiet der Medizintechnik und ist mit besonderem Vorteil in der Implantationsmedizin anwendbar. Es sind auch Anwendungen auf anderen Gebieten der Medizintechnik denkbar, bei denen eine gezielte Entkeimung und/oder Desinfektion von Gewebe wünschenswert ist.

In vielen Bereichen der Medizin, in denen ein Gewebe eines lebenden Patienten mit Fremdkörpern oder nicht körpereigenen Substanzen in Berührung kommt, ist die gezielte Entkeimung und/oder Desinfektion solcher Gegenstände oder Substanzen wünschenswert. Viele solche Stoffe oder Gegenstände können vor der Verwendung gründlich desinfiziert/sterilisiert werden. Es kann jedoch im Besonderen sinnvoll sein, eine Sterilisierung/Keimbeseitigung auch bei oder nach der Verwendung am Patientenkörper noch durchführen zu können. Beispielsweise kommt es bei Leitungen in Form von Kanülen oder elektrischen Leitungen, die durch die Haut oder ein Gewebe eines Patienten zwischen dem körperäußeren und dem körperinneren Bereich durchgeführt werden, im Übergangsbereich oder im körperinneren Bereich gelegentlich zu Infektionen. Diese können zu schwerwiegenden Komplikationen bei der Wundheilung und/oder beim Betrieb der entsprechenden Leitung führen. Solche Probleme können mit extern oder intern verabreichten Antibiotika behandelt werden; auf die Dauer führen solche Behandlungen jedoch zur Erzeugung von Resistenzen bzw. können solche Behandlungen bei multiresistenten Keimen bereits aussichtslos sein.

Der vorliegenden Erfindung liegt vor dem Hintergrund des Standes der Technik die Aufgabe zugrunde, eine effektive Desinfektionsmöglichkeit zu schaffen, die sowohl präventiv als auch während des Betriebs eines Gegenstandes oder einer Substanz aktiviert werden kann, die mit dem Gewebe eines Patienten in Kontakt gebracht wird. Dabei soll die Auswahl der behandlungsfähigen Keime möglichst groß sein, und es sollen keine neuen Resistenzen erzeugt werden.

Aus der US-Patentschrift US 5 904 646 A ist ein implantierter Aktuator bekannt, für den ein infektionsresistentes Kabel verwendet wird. Dort ist insbesondere ein Befestigungsmittel vorgesehen, das das Kabel mit einem Knochen des Patienten verbindet, um Bewegungen des Kabels relativ zum Gewebe des Patienten zu minimieren.

Die Aufgabe wird mit den Merkmalen der Neuerung gemäß Patentanspruch 1 gelöst. Vorteilhafte Ausgestaltungen der Neuerung sind den Unteransprüchen 2 bis 11 zu entnehmen. Der Patentanspruch 12 gibt ein Verfahren zur Anwendung der Neuerung an.

Demgemäß bezieht sich die Neuerung auf eine Einrichtung zur Durchführung einer Leitung durch die Haut eines Patienten. Die Aufgabe wird dadurch gelöst, dass die Leitung einen photodynamischen Stoff aufweist, der bei Bestrahlung hochreaktive Sauerstoffderivate freisetzt.

Als photodynamischer Stoff wird dabei ein solcher Stoff bezeichnet, der einen photosensiblen Stoff enthält, der durch Licht geeigneter Wellenlänge im optischen Bereich angeregt wird und in Anwesenheit von Sauerstoff reaktive Sauerstoffderivate erzeugt. Dies ist durch zwei photooxidative Prozesse möglich:

Erstens können durch einen photooxidativen Prozess Radikale entstehen, die mit Sauerstoff reagieren und Oxidationsprodukte bilden. Die Richtung des Elektronentransfers wird durch das Redoxpotential zwischen dem Photosensibilisator und Sauerstoff bestimmt.

Zweitens kann nach der Lichtanregung der photosensible Stoff Energie direkt auf Sauerstoffatome/Moleküle übertragen, wobei der hochreaktive Singulett-Sauerstoff entsteht, der unmittelbar mit entsprechenden Zielstrukturen in seiner nächsten Umgebung reagiert.

Als Photosensibilisator können Stoffe aus der chemischen Gruppe der Phenothiazine, Phthalocyanine oder Porphyrine verwendet werden, um sowohl multiresistente Gram-positive als auch Gram-negative Bakterien zu inaktivieren. Die entstehenden reaktiven bzw. hochreaktiven Sauerstoffderivate wirken stark desinfizierend/keimtötend.

Ein Vorteil der Neuerung liegt darin, dass der photosensitive Stoff (Photosensibilisator) gezielt durch Bestrahlung im optischen Bereich aktiviert werden kann. Dadurch ist eine aktive Keimbehandlung auch während des Einsatzes im Körper eines Patienten möglich, ohne weitere Schädigungen des organischen Gewebes zu riskieren. Auch der Ort der Einwirkung auf den Photosensibilisator kann durch entsprechende Ausrichtung der Bestrahlung selektiv gewählt werden. Der entsprechende hochreaktive Sauerstoff kann auch multi-resistente Keime, bei denen Antibiotika nicht mehr oder sehr schlecht wirken, inaktivieren.

Die Konzentration und/oder Verteilung des photosensitiven Stoffes kann auch so gestaltet werden, dass der Stoff mehrmals mit zeitlichem Abstand zur Freisetzung von hochreaktivem Sauerstoff bestrahlt werden kann. Es können auch gleichzeitig verschiedene photosensitive Stoffe eingebracht werden, die beispielsweise für verschiedene Bestrahlungs-Wellenlängen sensitiv sind, so dass die Einrichtung mehrmals verwendet werden kann.

Bei der Leitung, die den photodynamischen Stoff aufweist, kann es sich einerseits um eine elektrische Leitung handeln, die einen oder mehrere elektrische Leiter und eine Umhüllung der Leiter, beispielsweise in Form eines Kabelmantels, aufweist. Es kann sich bei der Leitung jedoch auch um eine oder mehrere Kanülen oder schlauchartige Elemente handeln, die jeweils Hohlräume zur Leitung von Flüssigkeiten oder Gasen aufweisen.

In beiden Fällen kann der photodynamische Stoff beispielsweise in der Umhüllung oder im Mantel der Leitung angeordnet sein, so dass die Umhüllung / der Mantel der Leitung wenigstens teilweise aus einem photodynamischen Stoff besteht oder mit einem photodynamischen Stoff beschichtet ist. Beispielweise kann es sein, dass dem Material der Umhüllung / des Mantels ein photodynamischer Stoff beigemischt ist oder dass makroskopische Einlagen aus einem photodynamischen Werkstoff dem Material der Umhüllung / des Mantels beigefügt sind. Beispielsweise können entlang der Leitung in den Mantel ein oder mehrere Stränge aus einem photodynamischen Stoff integriert sein.

Ebenfalls kann vorgesehen sein, dass ein Mantel der Leitung wenigstens auf einem Teilabschnitt seiner Länge ganz oder teilweise aus einem photodynamischen Stoff besteht. In diesem Fall kann auch bei einer im Patientenkörper befindlichen Leitung in dem photodynamischen Stoff durch einfache Bestrahlung mit geeignetem Licht, beispielsweise in einer vorbestimmten Wellenlänge oder einem vorbestimmten Wellenlängenbereich, der hochreaktive Sauerstoff erzeugt/freigesetzt werden, um die Keimtötung zu beginnen.

Dabei kann beispielsweise vorausgesetzt werden, dass auch bei einer eingewachsenen Leitung ein Teil des eingestrahlten Lichts das Gewebe / die Haut des Patienten durchleuchtet und zu Bereichen der Leitung gelangt, die unter der Hautoberfläche liegen, so dass auch dort der photodynamische Stoff aktiviert werden kann.

Ist eine Abdeckeinrichtung in Form einer Wundabdeckung am Ort des Leitungseintritts in das Gewebe des Patientenkörpers vorgesehen, so kann diese Abdeckeinrichtung/Manschette wenigstens teilweise für eine den photodynamischen Stoff aktivierende Strahlung durchlässig sein. Die in Rede stehende Strahlung kann im optischen Bereich vorliegen, der sensitive Wellenlängenbereich kann allerdings auch im Infrarot- oder im ultravioletten Bereich liegen.

Die Abdeckeinrichtung kann insgesamt für eine solche Strahlung durchscheinend gestaltet sein, sie kann jedoch zur Einstrahlung von Licht auch entsprechende Fenster aufweisen.

Der photosensible/photodynamische Werkstoff sollte derart gewählt sein, dass bei geeigneter Bestrahlung bei dem photodynamischen Stoff ein photooxidativer Prozess abläuft.

Ein weiteres Auswahlkriterium für einen photosensiblen/photodynamischen Stoff kann sein, dass bei dem photodynamischen Stoff Radikale entstehen, die mit Sauerstoff reagieren.

Es kann zudem vorgesehen sein, dass der photodynamische Stoff derart ausgebildet ist, dass er photonisch anregbar ist, wobei bei einer Abregung aus dem angeregten Zustand Energie unmittelbar auf Sauerstoff übertragbar ist und Singulett-Sauerstoff erzeugt wird.

Die Neuerung kann zudem dadurch implementiert werden, dass der photodynamische Stoff ein Phenothiazin, ein Phthalocyanin oder ein Porphyrin oder eine Mischung mit zwei oder drei dieser Komponenten ist.

Eine weitere Möglichkeit zur Umsetzung der Neuerung kann vorsehen, dass eine Lichtleiteinrichtung vorgesehen ist, die wenigstens abschnittsweise innerhalb der Leitung oder parallel zu dieser verläuft und aus der Licht zur Bestrahlung der Leitung auskoppelbar ist. In diesem Fall kann durch die Lichtleiteinrichtung in besonders einfacher Weise das Licht zur Aktivierung des photodynamischen Stoffes an den Ort geleitet werden, wo eine Keimbehandlung notwendig erscheint.

Die Lichtleiteinrichtung kann beispielsweise einen Lichtwellenleiter umfassen, der in die Leitung integriert oder parallel zu dieser geführt ist. An einer Einkoppelstelle kann mittels einer Lichtquelle, beispielsweise eines Lasers, Licht in den Lichtwellenleiter eingekoppelt werden, und das Licht kann entweder zum gegenüberliegenden Ende des Lichtwellenleiters geleitet oder im Verlauf des Lichtwellenleiters wenigstens teilweise ausgekoppelt werden. Zur Auskopplung können beispielsweise Inhomogenitäten im Randbereich des Lichtwellenleiters, wie Einkerbungen oder Aufrauungen, oder Krümmungen des Lichtleiters vorgesehen sein. Sinnvollerweise werden Inhomogenitäten in dem Bereich des Lichtwellenleiters entlang seiner Länge vorgesehen, in dem voraussichtlich die Aktivierung eines photodynamischen Stoffes sinnvoll sein kann. Das eingestrahlte Licht kann beispielsweise an dem der Lichtquelle gegenüberliegenden Ende des Lichtwellenleiters reflektiert werden, um die Verluste zu minimieren.

Wird eine Leitung mit einem optisch durchsichtigen Mantel verwendet, so kann eine Lichtleiteinrichtung auch innerhalb der Leitung verlegt werden, so dass sie den Mantel / die Umhüllung der Leitung von innen durchstrahlen kann.

Die Neuerung kann sich auch auf eine Implantateinrichtung mit einem Implantat beziehen, beispielsweise einer Herzpumpe oder einem anderen Aggregat, eine transkutane Leitung mit einer Einrichtung zur Durchführung, wie oben beschrieben, und einem außerhalb des Patientenkörpers an die Leitung angeschlossenen Funktionselement, wie beispielsweise einer Energieversorgung oder einer Datenverarbeitungseinrichtung. Ebenso kann außerhalb des Patientenkörpers eine Blutpumpe vorgesehen sein, die mittels Fluidleitungen mit einer oder mehreren implantierten Andockelementen an Blutgefäße und/oder Teile eines Patientenherzens angekoppelt ist.

Ein Verfahren zur Umsetzung der Neuerung kann beispielsweise vorsehen, dass eine Umhüllung der Leitung, die einen photodynamischen Stoff enthält, mit Licht bestrahlt wird. Hierzu kann eine Lichtquelle, die auf die zu desinfizierenden Bereiche gerichtet oder mit den zu desinfizierenden Bereichen mittels einer Lichtleiteinrichtung verbunden ist, eingeschaltet und betrieben werden. Die Lichtquelle wird dann nach Durchführung der Desinfektion ausgeschaltet oder auf andere Bereiche gerichtet.

Im Folgenden wird die Neuerung anhand von Figuren einer Zeichnung detaillierter beschrieben und nachfolgend erläutert. Dabei zeigt
- Fig. 1: schematisch eine implantierte Flerzpumpe, die mit einer Steuereinheit außerhalb des Patientenkörpers verbunden ist,
- Fig. 2: eine außerhalb des Patientenkörpers angeordnete Pumpe, die mit einem Patientenherzen verbunden ist,
- Fig. 3: eine erste Leitung im Querschnitt,
- Fig. 4: eine zweite Leitung im Querschnitt,
- Fig. 5: eine dritte Leitung im Querschnitt mit einem parallel dazu verlaufenden Lichtwellenleiter,
- Fig. 6: eine vierte Leitung im Querschnitt,
- Fig. 7: eine Ansicht eines Lichtwellenleiters mit einer Lichtquelle,
- Fig. 8: eine fünfte Leitung im Querschnitt,
- Fig. 9: eine Wundabdeckung sowie
- Fig. 10: eine Schemadarstellung des photodynamischen Effekts.

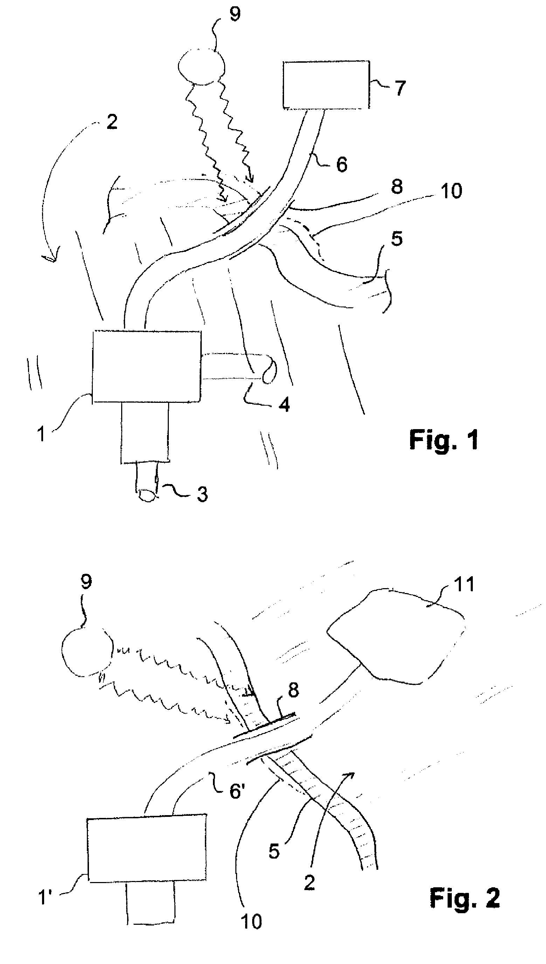

Figur 1 zeigt eine Herzpumpe 1, die in den Körper 2 eines Patienten implantiert ist. Der Pumpeneinlass 3 und der Pumpenauslass 4 sind nur der Vollständigkeit halber angedeutet, ohne dass der weitere Verlauf von Kanülen gezeigt ist.

Der Körper 2 des Patienten ist nach außen durch die Bauchdecke 5 abgeschlossen. Die Bauchdecke 5 wird durch eine Leitung 6 durchsetzt, die einen oder mehrere elektrische Leiter enthält, die einzeln oder gemeinsam von einem oder mehreren Kabelmänteln umgeben sind. Mittels der Leitung 6 ist die Pumpe 1 mit einer Steuereinheit 7 verbunden. Im Bereich der Durchführung durch die Bauchdecke 5 ist die Leitung 6 mit einer Beschichtung 8 aus einem photodynamischen Material versehen, die sowohl außerhalb des Patientenkörpers einen Teil der freiliegenden Leitung 6 bedeckt als auch innerhalb des Patientenkörpers einen Bereich der Leitung unterhalb der Bauchdecke 5.

Es ist zudem eine Lichtquelle 9 angedeutet, die Licht aussendet, so dass der photodynamische Werkstoff 8 bei Lichtabsorption hochreaktiven Sauerstoff zur Desinfektion im beschichteten Bereich der Durchführung der Leitung 6 freisetzt.

Die Beschichtung mit dem photodynamischen Werkstoff kann ebenso an der Außenseite der Bauchdecke 5 enden und ausschließlich im Inneren des Patientenkörpers vorgesehen sein.

Die Eintrittsöffnung durch die Bauchdecke 5 ist mittels einer Wundabdeckung 10 abgedeckt, die den Durchführungsbereich vor Umwelteinflüssen und Keimen schützen soll. Die Abdeckung 10 (gestrichelt dargestellt) kann derart ausgebildet sein, dass sie die Strahlung von der Lichtquelle 9 zu der Beschichtung 8 durchlässt. Die Wundabdeckung 10 kann ihrerseits auf ihrer dem Patientenkörper zugewandten Seite eine Beschichtung aus einem photodynamischen Material aufweisen, so dass bei entsprechender Lichtbestrahlung auch dort eine Desinfektion im Bereich der Haut des Patienten stattfinden kann.

In Figur 2 ist eine Pumpe 1' zur Herzunterstützung dargestellt, die außerhalb des Patientenkörpers 2 angeordnet und mit dem Herzen 11 des Patienten mittels einer Leitung 6' verbunden ist. Die Leitung 6' ist im Wesentlichen durch eine Kanüle gebildet, durch die Blut zwischen dem Herzen 11 und der Pumpe 1' gefördert wird. Die Leitung 6' durchsetzt die Bauchdecke 5 oder beispielsweise auch einen anderen Teil der Haut des Patienten und ist im Bereich der Durchführung mit der Beschichtung 8 aus einem photodynamischen Werkstoff versehen. Wie in Figur 1 ist eine Wundabdeckung 10 über der Durchtrittsöffnung der Leitung 6' gestrichelt dargestellt. Diese sollte für die Strahlung der Lichtquelle 9, die zur Aktivierung des photodynamischen Stoffes notwendig ist, durchlässig sein. Es ist jedoch auch denkbar, dass zur Bestrahlung die Wundabdeckung 10 entfernt wird.

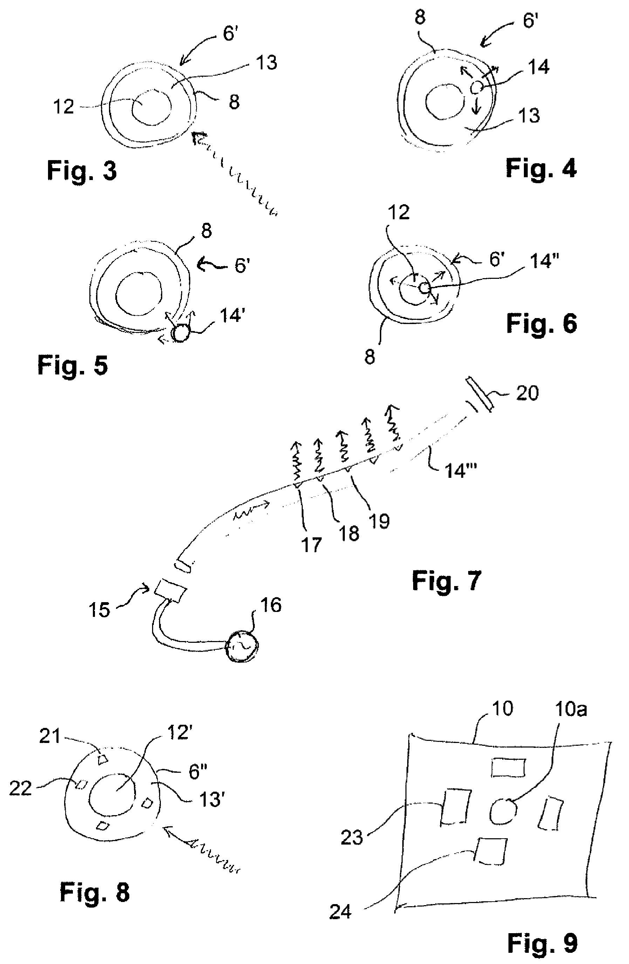

Figur 3 zeigt einen möglichen Querschnitt einer Leitung 6' mit einem zentrischen Hohlraum 12, einer Kanülenwand 13 und einer äußeren Beschichtung 8 aus einem photodynamischen Werkstoff.

Figur 4 zeigt im Querschnitt eine Kanüle ähnlich der aus Figur 3, wobei in die Wand 13 der Kanüle ein Lichtwellenleiter 14 integriert ist, der dazu eingerichtet ist, in radialer Richtung Licht auszusenden. Damit kann die Beschichtung 8 mit Licht aus dem Lichtwellenleiter 14 bestrahlt werden, um die photodynamische Wirkung zu erzeugen.

Figur 5 zeigt eine Kanüle 6' mit einem parallel zu dieser geführten Lichtwellenleiter 14', der beispielsweise außen an die Kanüle 6' geklebt sein kann. Der Lichtwellenleiter 14' strahlt radial Licht ab, das von der Beschichtung 8 aus einem photodynamischen Stoff absorbiert werden kann.

Figur 6 zeigt eine Kanüle 6', bei der im zentrischen Hohlraum 12 ein Lichtwellenleiter 14" geführt ist, der Licht radial abstrahlen kann, welches durch die photodynamische Schicht 8 absorbiert werden kann.

In Figur 7 ist ein Beispiel für einen Lichtwellenleiter dargestellt, der dazu eingerichtet ist, radial Licht abzustrahlen. Üblicherweise sind Lichtwellenleiter derart konstruiert, dass sie möglichst wenig Licht radial abstrahlen, sondern dieses ausschließlich axial und möglichst verlustfrei führen. Ergeben sich jedoch Bereiche großer Krümmung (eines kleinen Krümmungsradius) oder Einkerbungen oder Aufrauungen im Außenbereich an der Mantelfläche des Lichtwellenleiters 14"', so wird dort Licht radial ausgekoppelt und kann zur Bestrahlung einer parallel zu dem Lichtwellenleiter verlaufenden Kanüle benutzt werden. In Figur 7 ist mit 15 eine Lichtquelle, beispielsweise in Form eines Lasers, bezeichnet, die durch eine Energiequelle 16 gespeist ist. Durch den Laser wird Licht in den Lichtwellenleiter 14'" eingekoppelt und durch die - schematisch dargestellten - Kerben 17, 18, 19 radial ausgekoppelt. Um eine möglichst große Lichtausbeute für die radiale Abstrahlung zu erhalten, kann das axial durchgehende Licht an einem Spiegel 20 in den Lichtwellenleiter 14"' zurückreflektiert werden.

Figur 8 zeigt eine Kanüle 6" mit einem zentrischen Hohlraum 12', wobei die Wand 13' der Kanüle aus einem Kunststoff besteht, in den Partikel 21, 22 aus einem photodynamischen Stoff eingelagert sind. In diesem Fall kann auf eine Beschichtung der Kanüle 6" im Mantelbereich mit einem photodynamischen Werkstoff verzichtet werden. Voraussetzung dafür ist allerdings, dass die Wand 13" der Kanüle für den hochreaktiven Sauerstoff durchlässig ist und/oder dass die eingelagerten Partikel 21, 22 so weit im radial äußeren Bereich der Kanülenwand 13' liegen, dass der dort freigesetzte hochreaktive Sauerstoff an die Mantelfläche der Kanüle 6" gelangen kann.

In Figur 9 ist eine Draufsicht auf eine Wundabdeckung 10 dargestellt, die eine mittlere Öffnung 10a zur Durchführung der Leitung 6, 6', 6" sowie optische Fenster 23, 24 aufweist, die zur Bestrahlung des dahinterliegenden Bereichs der Kanüle mit Licht dienen, falls das Material der Wundabdeckung 10 an sich nicht für diese Strahlung durchlässig ist. Es kann jedoch auch die gesamte Abdeckung 10 aus einem für das Licht durchscheinenden Material bestehen, so dass der photodynamische Stoff durch die Wundabdeckung 10 hindurch aktiviert werden kann.

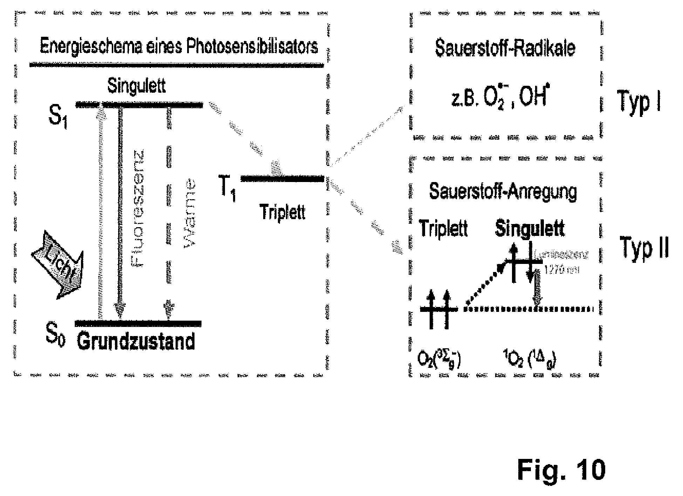

In Figur 10 ist schematisch das Energieschema eines photodynamischen Werkstoffs (Photosensibilisators) gezeigt. Wird der Photosensibilisator mit geeignetem Licht oder einer Strahlung geeigneter Wellenlänge bestrahlt, so wird Sauerstoff, der ein Bestandteil des photodynamischen Stoffes oder in seiner unmittelbaren Umgebung angeordnet ist, von einem Grundzustand in einen Singulett-Zustand (Singulett-Sauerstoff) verändert. Der Singulett-Sauerstoff kann durch Fluoreszenz unter Wärmeabgabe in den Grundzustand zurückfallen, jedoch auch in einen Triplett-Zustand (T₁). Von diesem Triplett-Zustand aus kann es in einer ersten (Typ I) Reaktionsform zu einem Ladungstransfer zwischen einem angeregten PS (Photosensibilisator) im T₁-Zustand und einem Substratmolekül kommen, wobei ein Elektron oder ein Wasserstoffatom übertragen wird. Es entstehen Superoxidanionen-Radikale oder Hydroxyl-Radikale.

Vom Triplett-Zustand T₁ aus kann auch eine zweite (Typ II) Reaktion mit einem direkten Direktübertrag vom angeregten PS auf Sauerstoff stattfinden. Dies führt zur Relaxation des Photosensibilisators, aber auch zur elektronischen Sauerstoffanregung vom Grundzustand (Triplett-Zustand) in den niedrigsten Singulett-Zustand. Dieser sehr reaktive Singulett-Zustand wird auch als Singulett-Sauerstoff bezeichnet. Beide Reaktionen, Typ I und Typ II, führen zur Freisetzung von Stoffen, die Keime sehr wirksam abtöten können.

Die Erfindung ist mit Vorteil in der Implantationsmedizin einsetzbar und dort nicht nur im Bereich der Durchführung von Leitungen, sondern auch bei allen Arten von Wechselwirkungen zwischen Fremdkörpern und einem Patientenkörper, bei denen Keime in den Körper eingetragen werden können. Beispielsweise ist es denkbar, einen Operationsfaden mit einem photodynamischen Werkstoff zu beschichten oder teilweise aus einem derartigen Werkstoff herzustellen. Dies kann eine eigenständige Erfindung darstellen, ebenso wie die Beschichtung von Prothesen im Zahntechnikbereich oder auch von Prothesen von Gliedmaßen, die wenigstens teilweise aus dem Körper eines Patienten herausragen.

## Patentansprüche

1. Einrichtung zur Durchführung einer Leitung (6, 6', 6") durch die Haut eines Patienten, **dadurch gekennzeichnet, dass** die Leitung einen photodynamischen Stoff (8) aufweist, der bei Bestrahlung hochreaktive Sauerstoffderivate freisetzt.

2. Einrichtung nach Anspruch 1, **dadurch gekennzeichnet, dass** ein Mantel der Leitung (6, 6', 6") wenigstens teilweise aus einem photodynamischen Stoff (8) besteht.

3. Einrichtung nach Anspruch 1 oder 2, **dadurch gekennzeichnet, dass** ein Mantel der Leitung (6, 6', 6") wenigstens auf einem Teilabschnitt seiner Länge ganz oder teilweise aus einem photodynamischen Stoff (8) besteht.

4. Einrichtung nach Anspruch 1, 2 oder 3, **dadurch gekennzeichnet, dass** eine Abdeckeinrichtung (10), insbesondere in Form einer mit der Leitung (6, 6', 6") verbundenen Manschette, vorgesehen ist, die für eine den photodynamischen Stoff aktivierende Strahlung durchlässig ist.

5. Einrichtung nach Anspruch 1 oder einem der folgenden, **dadurch gekennzeichnet, dass** bei geeigneter Bestrahlung bei dem photodynamischen Stoff ein photooxidativer Prozess abläuft.

6. Einrichtung nach Anspruch 5, **dadurch gekennzeichnet, dass** bei dem photodynamischen Stoff (8) Radikale entstehen, die mit Sauerstoff reagieren.

7. Einrichtung nach Anspruch 5 oder 6, **dadurch gekennzeichnet, dass** der photodynamische Stoff photonisch anregbar ist, wobei bei einer Abregung aus dem angeregten Zustand Energie unmittelbar auf Sauerstoff übertragbar ist und Singulett-Sauerstoff erzeugt wird.

8. Einrichtung nach Anspruch 1, oder einem der folgenden, **dadurch gekennzeichnet, dass** der photodynamische Stoff ein Phenothiazin, ein Phthalocyanin oder ein Porphyrin oder eine Mischung aus zwei oder drei dieser Komponenten ist.

9. Einrichtung nach Anspruch 1 oder einem der folgenden, **dadurch gekennzeichnet, dass** die Leitung (6, 6', 6") eine Kanüle zur Leitung von Fluiden ist.

10. Einrichtung nach Anspruch 1 oder einem der folgenden, **dadurch gekennzeichnet, dass** die Leitung (6, 6', 6") eine elektrische Leitung mit einem Kabelmantel ist.

11. Einrichtung nach Anspruch 1 oder einem der folgenden, **dadurch gekennzeichnet, dass** eine Lichtleiteinrichtung (14, 14', 14", 14'") vorgesehen ist, die wenigstens abschnittsweise innerhalb der Leitung (6, 6', 6") oder parallel zu dieser verläuft und aus der Licht zur Bestrahlung der Leitung auskoppelbar ist.

12. Verfahren zur Desinfektion einer durch die Haut eines Patienten durchgeführten Leitung (6, 6', 6"), **dadurch gekennzeichnet, dass** eine Umhüllung (13,13') der Leitung (6, 6') mit Licht bestrahlt wird.
